# EUROPEAN PATENT APPLICATION

(11) **EP 2 044 850 A1**
(43) Date of publication of application: **08.04.2009**
(21) Application number: 07018359.5
(22) Date of filing: 19.09.2007
(51) Int. Cl.: A23L 1/29, A23L 1/30, A61K 36/00, A61P 3/04

(54) **Method for altering the metabolism characteristic of food products**

(71) Applicant: Clara's ApS, 3140 Ålsgårde (DK)
(72) Inventor: Nordgaard, Annika, Burrild, 3140 Ålsgårde (DK); Hansen, Preben, Nordgaard, 3140 Ålsgårde (DK)
(74) Representative: Nordic Patent Service

(57) **Abstract**

A method for preparing or refining a balanced food product comprising starting with a food product with a content of building substances and adding a catalyst adapted to alter said building substances metabolism characteristic to resemble a metabolism characteristic of a energy substance.

## Description

The present application relates to a method for preparing or refining food products. In particular the application relates to a method for preparing or refining food products by altering their metabolism characteristics.

### BACKGROUND OF THE INVENTION

In today's society it is a general consensus that it is important to stay healthy and one aspect of this involves minding one's weight so as not to become overweight as this might lead to serious diseases and other maladies. Carbohydrates are one of the main contributing factors for the modern-age general obesity especially since it's a part of so many food products around us. Many of the products supposed to be healthy containing a high sugar content e.g. fruit juice which has a sugar content which is on the level of most sodas on the market.

In modern day society is also considered important to have a very thin or slim and fit body for cosmetic and a aesthetical purposes. This has led to that even persons not suffering from obesity commonly go through various slimming exercises or procedures to improve their physical appearance. It is not uncommon for many to undergo extreme slimming procedures such as a surgery or intake of dangerous substances like amphetamine-based diet pills. Thus many of the available dieting procedures serve to upset the balance of a person's food intake in a way that is not always healthy.

Food products can be divided into three major groups, which are listed below in table 1, according to their function in the body.

**TABLE 2**

| | |
|---|---|
| Energy substances | Substances that produces energy to keep the body warm and to allow the organs to work properly. The energy has a high burn rate. |
| Building substances | Substances that produces building blocks or energy that can be stored in the cells, ie indirect energy which has a low burn rate. This is done on a cellular level by renewing the cells and any excess fat is stored in the cells. |
| Filler substances | Substances that are difficult to turn into energy, but can have other qualities such as beneficial for the vowel function. |

In table 2 the two groups of main concern are shown along with some common examples of food products that are rich in the various substances.

**TABLE 2**

| | |
|---|---|
| Building substances | Energy substances |
| Milk products, Barley products (pasta, bread, white rice) Juice, Soda, Candy Vegetable oils | Potatoes Sticky rice Coffee and Tea Fats Fish and meat Eggs |

It is important to keep a balance between these two groups to maintain a healthy diet. A more complete listing and description of these two food groups is to be found in "Fünf Elemente Ernärung" by Med Dr Ilse-Maria Fahrnow and Jürgen Fahrnow, published in 2005 by Gräfe und Unzer Verlag, GmbH.

The building substances commonly consists of carbohydrates, proteins (non-fat fish) and fats (butter and vegetable oils) and produce energy in a form that is difficult to burn ie has a low burn rate and is thus easily built up in the body and leads to a gain in body fat.

The energy substances commonly consists of proteins and fats (eggs, meat and fat fish) and produce energy in a form that can easily be burnt ie has a high burn rate and utilized by the body. The burn rate of these substances and the building substances also depend on outer circumstances such as availability as is commonly known.

Sometimes the building substances are referred to as being cold substances as the energy generated when digesting them does not heat the body and the energy substances are analogously referred to as warm substances as the energy generated through digestion is easily assimilated and thus heats the body.

If the person eats a lot of building substances but does not burn the calories or substances they will be turned into residual energy in the form of fat and the person will become fatter or overweight. In today's society most of the available food products are high in content of building substances. This is due to a number of processes like sweetening and refining where the original structure of the food product is c one hanged. However, the original structure and food products may well have a lower content of building substances but these might be unsuitable for further processing or consumption.

Thus there exist a need to reduce the amount of food products having a high content of building substances and also to restore the balance between the building substances and the energy substances.

### DISCLOSURE OF THE INVENTION

On this background, it is an object of the present application to provide a method that overcomes or at least reduces the drawbacks indicated above.

Realizing that, in addition to the three food groups discussed above there also exists a fourth group, namely the catalysts. These catalysts usually consists of proteins and either functions as a catalyst, that increase the metabolism and thereby alters the digestion characteristic of the building substances as the energy generated is burnt more easily due to the catalyst, or as hormones which also increase the metabolism. This insight makes it possible to change the metabolism characteristic of a building substance from being generating an energy that is difficult to burn or use (low burn rate) to resemble the energy substances characteristic of producing energy that is easy to burn (high burn rate). The energy, which is difficult to burn, generated by the building substances is reduced which thereby lowers the requirement for rigorous exercise to burn all the energy. This would enable a person eating food products with these added catalysts to loose weight and thus use the food products as slimming food products.

A consequence of the insight is that adding a small amount of these catalysts to food products can help restore the balance between the energy substances and the building substances. And this could thus be done without any major change to either the refining of sweetening processes or our general eating habits.

By realizing that adding one of these catalysts to a food product may cause its metabolism characteristics to resemble the building structures instead of its original building substance characteristics and thus behaving as building substance which restores the balance of a food product is crucial to the teachings of this application.

This object is achieved by providing a method for preparing or refining a balanced food product comprising starting with a food product with a content of building substances and adding a catalyst adapted to alter said building substances metabolism characteristic to resemble a metabolism characteristic of a energy substance to resemble a metabolism characteristic of a energy substance so as to increase the burn rate of energy generated. This provides for a food product that is more balanced having a slimming characteristic. This is done without addition of hazardous chemicals and can be done purely organic and ecologically.

A method may further be used on a food product that has a building substance content that is higher than said food product's content of energy substances whereupon a balance between the two substances is achieved upon the alteration of the metabolism characteristics of the building substance. This would indeed help bring the food products substances in balance.

A method may further be used on one of the following: Bread, Fruits, Vegetables, Flour, White sugar, Fats and Milk products.

A method may further have catalyst that is a spice and which spice is organic. The spice can be one of the following: Chili, Coriander, Turmeric, Cumin, Ginger and Onion.

The object is also met by a food product generated or refined through a method as described above.

Such a food product finds particular use as a slimming product and also for non-therapeutic or cosmetic slimming uses.

The object of this application is also met through a method for weight reduction comprising administering a food product as above to a patient not suffering from obesity or to an animal suffering from obesity. This would induce a cosmetic weight loss in the human and a therapeutic weight loss in the animal suffering from obesity or being over-weight.

An object of this application is also met through a catalyst adapted to alter a metabolism characteristic of a food product having building substances to wherein said metabolism characteristic is altered to resemble a metabolism characteristic of a energy substance. This would help restore a balance between the substances of a food product.

This catalyst may be organic which is good for both the individual eating the resulting food product and the environment.

It would also be possible to use a synthetic catalyst. Using a synthetic catalyst would make it possible to efficiently target a substance and/or to refine the catalyst into a purer and more efficient form.

An object of this application is also met through a use of a catalyst as above to prepare or refine a slimming food product.

Another object of the present application is the use of a method according to above for producing food products for human consumption.

Another object of the present application is to provide a food product produced through a method according the method described above as this food product is healthy without having given rise to hazardous byproducts and has a high quality. These food products could advantageously be used as for the non-therapeutic purpose of weight control, dieting or slimming, ie as a slimming food product. This finds particular use in cosmetic slimming.

Another object of the present application is to use a food product produced through a method as described above in the refining, preparation or manufacture of food products for human consumption. It should be noted that the food products need not be complete food products ready for consumption, but could also be for ingredients or sets that will later be used to produce food products for human consumption and also to produce or refine food products to be used as slimming food products.

Further objects, features, advantages and properties of the touchpad, method and computer readable medium according to the invention will become apparent from the detailed description.

### BRIEF DESCRIPTION OF THE DRAWINGS

In the following detailed portion of the present description, the teachings of the present application will be explained in more detail with reference to the exemplary embodiments shown in the drawings, in which:
Fig. 1 is a flow chart of a method according to an embodiment of the present application,
Fig. 2 is a block diagram showing the principle of the present application.

### DETAILED DESCRIPTION OF PREFERRED EMBODIMENTS

In the following detailed description, a method and a product according to the teachings of this application in the form of a process will be described by the embodiments.

A food product has a metabolism characteristic which describes how the food product is digested i.e. what it turns into and what it is used for as described earlier in the background section.

By adding a third type of substance here after called a catalyst it is possible to alter the metabolism characteristic of a substance in a food product. A special group of catalysts can alter the energy giving substances into generating an energy that is easy to burn thus increasing the burn rate. The balance between substances generating energies, that are easy respectively difficult to burn, in the food product is thereby changed.

One naturally occurring example of this is honey. Normal white sugar is clearly a building substance, but honey is not even though it is rich in sugar content. It is a energy substance. The reason it can be a energy substance when it is so high in energy is that it contains approximately 0.3 % of a protein that acts as a catalyst when digesting the sugar contained in the honey.

A commonly used ingredient in cooking is spices. Many spices have these catalyst properties and are thus well-suited for altering a food product into a slimming product. Some examples are: Chili, Coriander, Turmeric, Cumin, Ginger and Onion.

Spices are known to be added to food products to enhance the flavor, either simply for the tasting experience or to hide some other tastes such as that of meat going bad. However, adding one or more of these spices to a food product rich in building substances, does not only preserve its edibility as in the old days when the meat got bad etc, but also alters the metabolism characteristics of the food product. The new metabolism characteristics help restore the balance of the food product making it less fattening and in fact the food product turns into a slimming product. This is thus done without adding any dangerous chemicals and by preserving a well-tasting flavor of the food product.

It would also be possible to use a synthetic catalyst.

Products well-suited for this treatment are:
Bread, Fruit and vegetables, Flour, White sugar, Fats and Milk products.

When preparing a food product the catalyst can be added at any time, but the result is better if the catalyst is added at an early stage so as to give an opportunity to blend and meld better with the other substances.

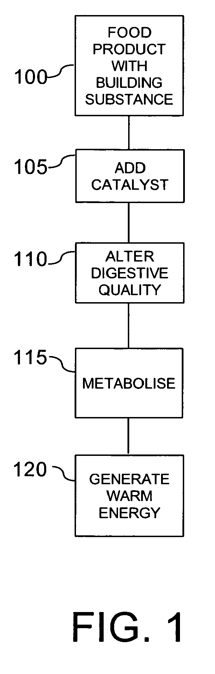

Figure 1 shows a flowchart according to this application where a food product with building substances, preferably quite high in building substances, are introduced to the method in step 100. A catalyst is added and allowed to meld with the food product in step 105. This catalyst changes the metabolism characteristics of the building substances and thereby also for the food product as is indicated in step 110. As the food product is digested in step 115 energy is generated in step 120, where the energy is of a kind that is easy to burn also called warm energy. The term warm is because it is said to warm the body.

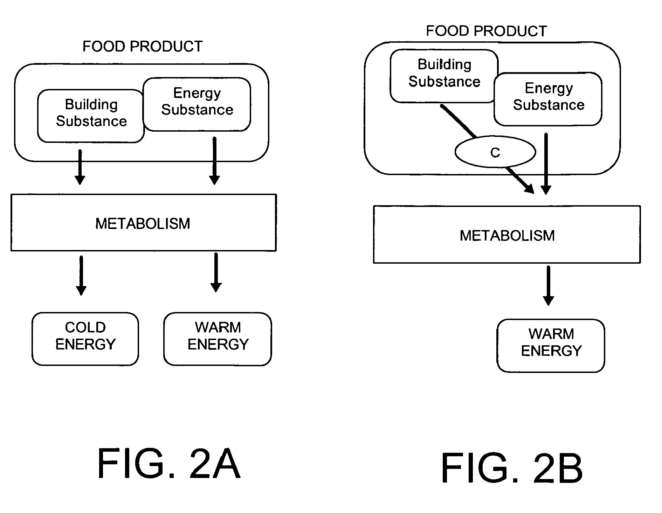

Figure 2 shows a block diagram of the effects of adding a catalyst. In fig 2A a food product consisting of Building substances and Energy substances are passed through a metabolism system. Here the substances are digested generating energy that is difficult to burn, or cold energy, from the building substances and energy that is easily burnt, or warm energy, from the energy substances. In fig 2B a catalyst C has been added to the food product. The catalyst is adapted to alter the metabolism characteristics of the building substance so when the food product passes through the metabolism system (DIGESTION) the building substances behave like the energy substances and only energy that is easy to burn, or warm energy, is generated.

For a new building substance not previously used, a dietrist could easily find which catalyst to use by testing which catalyst give the best alteration of the metabolism characteristic and then in which amount it is best tasting. For some catalyst perhaps an alternative having a lower yield is sometimes a better choice as the other catalyst might have other negative influences on the food product to be altered. One example could be to use turmeric instead of chili for a milk or bread product.

Through this process the food product quality is improved, from a health perspective. This is of utmost importance as the food products is to be used as food for humans and that requires a high level of quality unlike when, for example producing fodder products for animals or lifestock.

Again, it is imperative that no bad side effects are produced as the products are to be used for human consumption.

The teachings of this application find particular use in the non-therapeutic field of weight control or in other words for dieting or slimming products. As building substances generates a lot of energy that can be difficult to burn they tend to counteract a weight loosing or slimming activity. A food product such as described above have excellent slimming characteristics as it generates mainly warm energy, or energy that is easily burnt, and contains no hazardous byproduct.

When spices are used as catalysts the resulting slimming food product is in fact both ecological and organic assuming that the adopting food product was.

The above described method used for altering the metabolism characteristic of a food product could thus also be used to make normal food more adapted for slimming purposes or weight loss. In these cases the resulting food product would be a slimming food product. The method finds use both in the original generation of the food product and also to a later refinement of the food product to make them more slimming.

It should be understood that the method as described can also be applied to partial products, ie products that will be used as ingredients in a complete food product or a food product set to be used to later produce a complete food product. It should also be noted that the step of preparing, producing or refining a food product according to the teachings herein are all possible.

The method, the use of the method and any resulting food products could also be used to treat animals, pet or farm, suffering from obesity. This treatment would be ecological and economical as the fodder wouldn't need to be thrown away or otherwise replaced. A simple addition of the catalyst would suffice.

The term "comprising" as used in the claims does not exclude other elements or steps. The term "a" or "an" as used in the claims does not exclude a plurality. The single processor or other unit may fulfill the functions of several means recited in the claims.

## Claims

1. A method for preparing or refining a balanced food product comprising starting with a food product with a content of building substances and adding a catalyst adapted to alter said building substances metabolism characteristic to resemble a metabolism characteristic of a energy substance so as to increase the burn rate of energy generated.

2. A method according to claim 1, wherein said food product has a building substance content that is higher than said food product's content of energy substances whereupon a balance between the two substances is achieved upon the alteration of the metabolism characteristics of the building substance.

3. A method according to claim 1 or 2, wherein said food product is one of the following: Bread, Fruits, Vegetables, Flour, White sugar, Fats and Milk products.

4. A method according to claim 3, wherein said catalyst is a spice being organic.

5. A method according to claim 4, wherein said spice is one of the following: Chili, Coriander, Turmeric, Cumin, Ginger and Onion.

6. A method according to claim 3, wherein said catalyst is synthetic.

7. A food product generated or refined through a method as in any of claims 1 - 6.

8. A food product as in claim 7, wherein said food product is a slimming product.

9. A food product as in claim 7, wherein said slimming food product is for non-therapeutic uses.

10. A method for weight reduction comprising administering a food product as in claim 5, 6, 7, 8 or 9 to a patient not suffering from obesity.

11. A method for weight reduction comprising administering a food product as in claim 5, 6, 7, 8 or 9 to an animal suffering from obesity.

12. A catalyst adapted to alter a metabolism characteristic of a food product having building substances to wherein said metabolism characteristic is altered to resemble a metabolism characteristic of a energy substance.

13. A catalyst according to claim 12 which is organic.

14. Use of a catalyst according to claim 12 or 13 to prepare or refine a slimming food product.
